# EUROPEAN PATENT APPLICATION

(11) **EP 3 617 223 A1**
(43) Date of publication of application: **04.03.2020**
(21) Application number: 18770219.6
(22) Date of filing: 02.03.2018
(51) Int. Cl.: C07K 14/46, C12N 15/12, A61K 38/17, A61K 47/60

(54) **APPLICATION OF SELECTIVE TNFR1 ANTAGONIST PEPTIDE SN10 IN PREPARATION OF DRUGS FOR PREVENTING AND TREATING RHEUMATOID ARTHRITIS**

(30) Priority: 23.03.2017 CN 201710178435
(71) Applicant: Guilin Eight Plus One Pharmaceutical Co., Ltd., Guilin, Guangxi 541000 (CN)
(72) Inventor: LU, Yiming, Shanghai 200433 (CN); WANG, Jie, Shanghai 200433 (CN); LI, An, Shanghai 200433 (CN); JIANG, Hailong, Shanghai 200433 (CN); BIAN, Yingying, Shanghai 200433 (CN); ZHANG, Chuan, Shanghai 200433 (CN)
(74) Representative: Keil & Schaafhausen Patent- und Rechtsanwälte PartGmbB
(86) International application number: PCT/CN2018/077862
(87) International publication number: WO 2018/171406

(57) **Abstract**

The invention relates to the field of biomedicine, in particular to providing a selective TNFR1 antagonist peptide derived from the snake venom of Qinghai Hydrostatin-SN10 has the amino acid sequence as shown in SEQ ID NO: 2.The present invention also provides a selective TNFR1 antagonist peptide PEG-SN10 based on m PEG2000 modification, which is selectively modified by covalent attachment of the carboxyl group of m PEG2000 to the free amino group of the N-terminal aspartic acid of Hydrostatin-SN10 peptide chain. At the same time, the present invention provides the use of the selective TNFR1 antagonist peptides Hydrostatin-SN10 and PEG-SN10 for the prevention and treatment of rheumatoid arthritis.

## Description

### Technical Field

The invention relates to the field of biomedicine technology, in particular, is a selective TNFR1 antagonistic peptide Hydrostatin-SN10 derived from the venom of *Hydrophis cyanocinctus* and its application in rheumatoid arthritis.

### Technical Background

Rheumatoid arthritis (RA) is a chronic autoimmune disease characterized by joint synovitis. The main clinical manifestations are joint swelling and pain caused by synovitis, erosion of cartilage and bone caused by inflammation and hyperplasia in synovial membrane, narrowing of joint space, and joint rigidity, deformity, dysfunction caused by severe bone destruction and absorption in the late stage. In the United States, RA is considered to be one of the five major diseases affecting human health (Cardiovascular Disease, Alzheimer's disease, Cancer, AIDS, and Rheumatoid Arthritis). RA occur at any age, with a maximum at 20-50 years old. Around 0.24 percent of people worldwide are suffering from RA, with a very high rate of disability and poor prognosis (Cross, M., Smith, E., Hoy, D., Carmona, L., Wolfe, F., Vos, T., Williams, B., Gabriel, S., Lassere, M., Johns, N., Buchbinder, R., Woolf, A., March, L., 2014. The global burden of rheumatoid arthritis: estimates from the global burden of disease 2010 study. Ann. Rheum. Dis. 73, 1316-132.).

So far, the pathogenesis of RA is not clear, but the genetic and environmental factors are closely related to the development of disease. Studies have shown that the severity of rheumatoid arthritis is significantly affected by inflammatory mediators. The main inflammatory mediators are tumor necrosis factor-α (TNF-α), interleukin-6 (IL-6), interleukin-17 (IL-17) and interleukin-1 (IL-1), etc., in which, TNF-α plays a very important role in pathogenesis of RA. Currently, drugs against TNF-α such as adamuzumab and infliximab have achieved good clinical effects in the clinical treatment of rheumatoid arthritis, which can not only improve symptoms but inhibit joint damage. However, these kind of anti-TNF-a monoclonal antibodies was completely blocked the biological function of TNF-α, affecting the immune homeostasis and immune surveillance function, which makes patients take new risks of tuberculosis infection, new autoimmune diseases and even tumors.

Excessive expression of TNF-α and abnormal activation of TNF-α/TNFRs signaling pathway are closely related to the occurrence and development of autoimmune diseases such as rheumatoid arthritis. As the research of the pathogenesis of RA moves along, more research have focused on TNFRs. However, relative to anti-TNF-a monoclonal antibodies, the basic and applied research of small molecule antagonists of TNFRs developed slowly. The overall From the perspective of anti-inflammation, TNFR1 mainly transmits pro-inflammatory and apoptotic signals. Selectively blocking the TNFR1 signal pathway to block the biological function of TNF-α has become the hot spots of these drugs. Currently, there is not a highly selective TNFR1 antagonist for clinical application.

On the basis of the previous work, our research group selected the phage display library of the venom of *Hydrophis cyanocinctus* for panning, and obtained an anti-inflammatory activity peptide Hydrostatin-SN1(Chinese patent literature CN103030687A). Then we obtained the candidate peptide Hydrostatin-SN10 by structural optimization. BIAcore(Bio-macromolecular Interaction Analysis based on Surface Plasmon Resonance) and MST(Micro-thermophoresis) were used to analyze the interaction between Hydrostatin-SN10 and TNF-α, TNFR1 and TNFR2, which demonstrated Hydrostatin-SN10 directly bind to TNFR1 but not to TNF-α or TNFR2. Hydrostatin-SN10 is a selective TNFR1 antagonistic peptide with specific target. Patent Literature CN103030687A published Hydrostatin-SN1 was able to treat diseases associated with TNF-α, which was rheumatoid arthritis. According to the mechanism of the earlier application, it can't be clearly derived that Hydrostatin-SN10 can be used to treat rheumatoid arthritis.

However, the small molecular polypeptides, which have low molecular weight, are rapid filtered by glomeruli and degraded by associated protease, resulted in poor stability, short half-life of plasma, unnormal biological function, and ineffective treatment, which restricts its development and application to some extent. In order to solve this problem, modifying the polypeptide drug or fusing with other materials to improve its stability has become a hot spot in bio-pharmaceutical research. Currently, there are a lot of chemical modifier used for peptides and proteins, methoxy-Polyethylene Glycol(mPEG) and its derivative is one of the most widely used modifiers. The advantages of PEG modification mainly include: 1, improving pharmacokinetic properties and extending the half-life of plasma; 2, increase solubility and stability (thermal stability, acid-base resistance, denaturant resistance and antiprotease hydrolysis);3, reduce immunogenicity and toxicity; 4, improve the biological activity in the body. There are several polyglycol modified proteins and peptides are approved for clinical use, such as PEG-TNF-α antibody Fab fragment, PEG-erythropoiesis polypeptide and so on. Hence, we modified Hydrostatin-SN10 based on the previous work and obtained PEG-SN10. This invention investigate the role of PEG-SN10 in rheumatoid arthritis.

### Contents of the invention

The invention aims to provide a selective TNFR1 antagonistic peptide Hydrostatin-SN10 derived from the venom of *Hydrophis cyanocinctus* and its application in rheumatoid arthritis. Another purpose of the invention is to provide a selective TNFR1 antagonistic peptide Hydrostatin-SN10 modified by methoxy-Polyethylene Glycol 2000(mPEG2000), namely PEG-SN10 and its application in rheumatoid arthritis. Inventor's research group (Hailong Jiang, 2015, Master degree dissertation of the second military medical university of the people's liberation army, the structure optimization and anti-inflammatory mechanism of Hydrostatin-SN1 from sea snake) obtained Hydrostatin-SN10 (10AA) by truncating Hydrostatin-SN1 (22AA). Surface plasmon resonance (SPR) demonstrated the binding capacity of Hydrostatin-SN10 with TNFR1 and the binding capacity was approximately KD=2.8µM, higher than the binding capacity of Hydrostatin-SN1 with TNFR1 (KD=32µM). However, it is not clear whether Hydrostatin-SN10 is selectively antagonistic with TNFR1. The results of animal model show that it has certain anti-inflammatory activity.

Main technical scheme of the invention: establishing the rheumatoid arthritis animal models induced by type II bovine collagen to demonstrate Hydrostatin-SN10 and PEG-SN10 can be used to treat rheumatoid arthritis.

The first aspect of the invention provides a selective TNFR1 antagonist peptide Hydrostatin-SN10, the amino acid sequences of the selective TNFR1 antagonistic peptide Hydrostatin-SN10 mentioned here are shown as SEQ ID NO:2.

The synthetic method of the selective TNFR1 antagonistic peptide Hydrostatin-SN10 mentioned here is synthesizing Hydrostatin-SN10 by solid phase synthesis, analyzing the purity and molecular weight by HPLC and MS. Molecular weight was 1250.29 Dalton and the isoelectric point was 4.39.

The second aspect of the invention provides the encoding gene of a selective TNFR1 antagonist peptide Hydrostatin-SN10, its nucleotide sequence is shown as SEQ ID NO:1.

The third aspect of the invention provides the application of selective TNFR1 antagonist peptide Hydrostatin-SN10 mentioned here in the preparation of medicines for the prevention and treatment of rheumatoid arthritis.

Optimized and mentioned medicine for prevention and treatment of rheumatoid arthritis is the pharmaceutical composition which the selective TNFR1 antagonistic peptide Hydrostatin-SN10 is the only active ingredient, or contains selective TNFR1 antagonistic peptide Hydrostatin-SN10.

The fourth aspect of the invention provides a mPEG2000(methoxy-Polyethylene Glycol 2000) modified selective TNFR1 antagonistic peptide Hydrostatin-SN10, namely PEG-SN10. Carboxyl of the mPEG2000 mentioned here connects to the free amino group of aspartic acid in the N-terminal of Hydrostatin-SN10. The half-life and stability of Hydrostatin-SN10 modified by mPEG2000(methoxy-Polyethylene Glycol 2000) with an average molecular weight of about 2000 Dalton are increased. The simplified structure of mPEG2000 can be expressed as: CH₃O-(CH₂CH₂O)ₙ-COOH, where the n is polymerization degree. The average molecular weight is 2000 Dalton. The fifth aspect of the invention provides the application of selective TNFR1 antagonist peptide Hydrostatin-SN10 modified by mPEG2000 in the preparation of medicines for the prevention and treatment of rheumatoid arthritis.

Optimized and mentioned medicine for prevention and treatment of rheumatoid arthritis is the pharmaceutical composition which PEG-SN10 is the only active ingredient, or contains PEG-SN10.

Optimized and mentioned pharmaceutical composition and conventional pharmaceutical excipients are prepared into pharmaceutical preparation.

Optimized and mentioned pharmaceutical preparation is tablet, granule, dispersant, capsule, soft capsule, drop pill, injection, powder, aerosol and so on.

Optimized and mentioned medicine for prevention and treatment of rheumatoid arthritis selectively antagonize TNFR1.

In this invention, BIAcore (Bio-macromolecular Interaction Analysis based on Surface Plasmon Resonance) and MST(Micro-thermophoresis) were used to analyze the interaction between Hydrostatin-SN10 and TNF-α, TNFR1 and TNFR2, which demonstrated Hydrostatin-SN10 directly bind to TNFR1 but not to TNF-α or TNFR2. The invention used rheumatoid arthritis animal models induced by type II bovine collagen to investigate the treatment effect of the peptide mentioned here. The results showed Hydrostatin-SN10 and PEG-SN10 were effective in reducing Arthritis index in CIA mice after intraperitoneal administration of Hydrostatin-SN10 and PEG-SN10. Hydrostatin-SN10 and PEG-SN10 significantly reduced the level of collagen specific antibody IgG and pro-inflammatory factors(IL-17, TNF-α, IL-6 and IFN-γ), increased the level of anti-inflammatory factor(IL-10) in serum. Hydrostatin-SN10 and PEG-SN10 plays an anti-inflammatory role through regulating the inflammatory factors. Micro-CT scan show that Hydrostatin-SN10 and PEG-SN10 significantly improve the degree of bone destruction in bone trabecula. Hydrostatin-SN10 and PEG-SN10 have anti-inflammatory effects from bone destruction. Hydrostatin-SN10 and PEG-SN10 significantly improved joint injury, synovial hyperplasia and inflammatory cell infiltration and reduced the expression of TNF-α and the number of osteoclasts in joints. Hydrostatin-SN10 and PEG-SN10 have anti-inflammatory effects from joint pathology. Hydrostatin-SN10 and PEG-SN10 regulated ratio imbalance of Th1/Th2 and Th17/Treg, namely Hydrostatin-SN10 and PEG-SN10 play a role against rheumatoid arthritis through immune regulation.

The above results indicated that selective TNFR1 antagonist Hydrostatin-SN10 and PEG-SN10 have good treatment ability of rheumatoid arthritis. The invention finds a medicine which effectively prevent and treat the rheumatoid arthritis.

### Description of the figures

Figure 1 HPLC analysis results of Hydrostatin-SN10.
Figure 2 MS analysis results of Hydrostatin-SN10.
Figure 3 BIAcore (SPR technology) was used to analysis of the binding capacity of Hydrostatin-SN10 and TNFR1. A) the interaction between SN10 and TNFR1, the dissociation constant K_{D} was about 2.8µM; B) the interaction between SN10 and TNF-α, no binding; C) Competitive inhibition of SN10 between TNF-α and TNFR1 (TNFR1 on the chip); D) Competitive inhibition of SN10 between TNF-α and TNFR1 (TNF-α on the chip); E) Competitive inhibition of SN10 between TNF-α and TNFR2.
Figure 4 MST was used to analysis of the binding capacity of Hydrostatin-SN10 and TNFR1. A) the interaction between SN10 and TNFR1, the dissociation constant K_{D} was about 2.8µM; B) the interaction between SN10 and TNF-α, no binding; C) the interaction between SN10 and TNFR2, no binding; D) Competitive inhibition of SN10 between TNF-α and TNFR1 (TNFR1 fluorescent labeling); E) Competitive inhibition of SN10 between TNF-α and TNFR1(TNF-α fluorescent labeling); F) Competitive inhibition of SN10 between TNF-α and TNFR2(TNFR2 fluorescent labeling).
Figure 5 Hydrostatin-SN10 and PEG-SN10 improve the volar swelling of CIA mice.
Figure 6 Hydrostatin-SN10 and PEG-SN10 improve the clinical scores of CIA mice.
Figure 7 Hydrostatin-SN10 and PEG-SN10 effect the collagen-specific antibody IgG and inflammatory factors in serum of CIA mice(n=6).
Figure 8 Hydrostatin-SN10 and PEG-SN10 effect the hindlimb joints of CIA mice (n=12).
Figure 9 Hydrostatin-SN10 and PEG-SN10 effect the bone trabeculae in CIA mice (n=12).
Figure 10 Hydrostatin-SN10 and PEG-SN10 effect the bone trabecula parameters in CIAmice(n=12).
Figure 11 Hydrostatin-SN10 and PEG-SN10 effect the ankle lesion in CIA mice. HE staining light microscopy of the tissue section, A) Normal group; B) Model group; C) Hydrostatin-SN10; D) PEG-SN10; E) Infliximab group.
Figure 12 Hydrostatin-SN10 and PEG-SN10 effect the expression level of TNF-α in CIA mice, A) Normal group; B) Model group; C) Hydrostatin-SN10; D) PEG-SN10; E) Infliximab group.
Figure 13 Hydrostatin-SN10 and PEG-SN10 effect the expression level of TRAP in CIA mice, A) Normal group; B) Model group; C) Hydrostatin-SN10; D) PEG-SN10; E) Infliximab group.
Figure 14 Hydrostatin-SN10 and PEG-SN10 effect the expression level of Foxp3 in Treg cells of CIA mice (n=6).
Figure 15 Hydrostatin-SN10 and PEG-SN10 effect the expression level of Foxp3 in Treg cells of CIA mice (n=6).
Figure 16 Hydrostatin-SN10 and PEG-SN10 effect the proportion of Th1, Th2, Th17 in the T cells of CIA mice(n=6).
Figure 17 Hydrostatin-SN10 and PEG-SN10 effect the proportion of Th1, Th2, Th17 in the T cells of CIA mice(n=6).

### Detailed description

The specific description will be described in detail examples.

The experimental methods in the following examples are conventional methods unless otherwise specified.

Hydrostatin-SN10 prepared in Example 1 was used in the experiments of Examples 2-5. The PEG-SN10 used in the following examples was synthesized by Qiang Yao Biotechnology Co., Ltd., and the purity was ≥98% by HPLC.

### Example 1: Synthesis of a selective TNFR1 antagonist peptide Hydrostatin-SN10

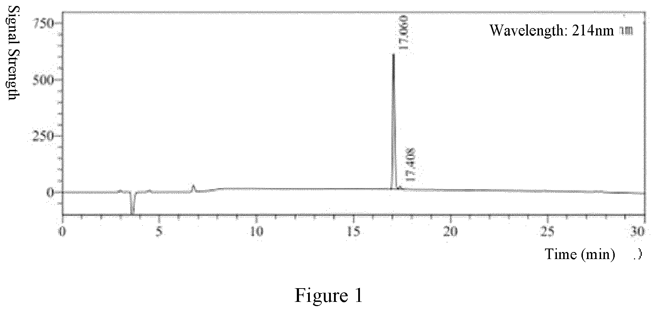

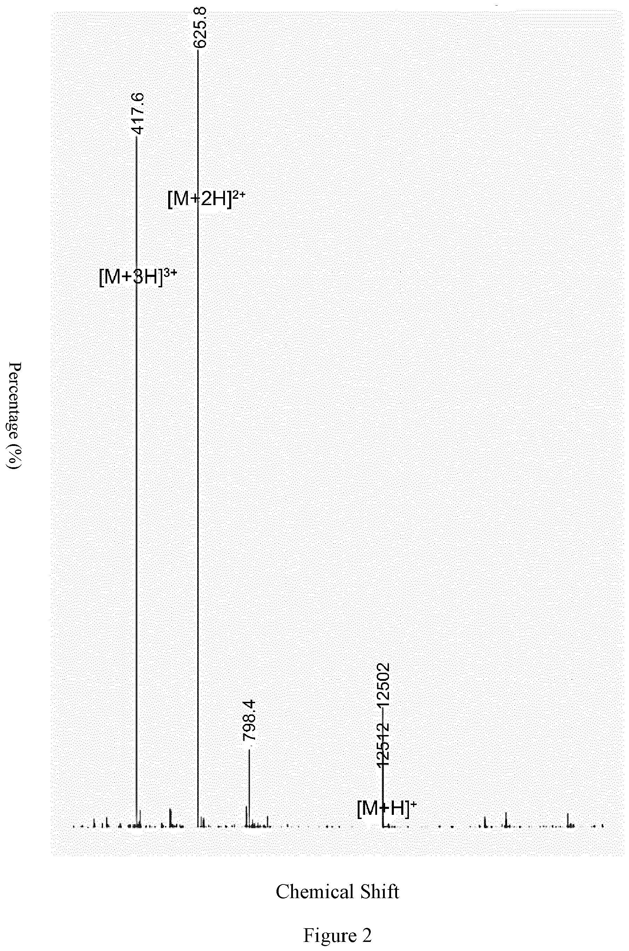

Hydrostatin-SN10 was synthesized by solid phase peptide synthesis, and its purity and molecular weight were analyzed by HPLC (Fig. 1) and MS (Fig. 2). The results showed that the purity was >97% and the molecular weight was 1250.29g/mol.

### Example 2: BIAcore analysis of the binding capacity of Hydrostatin-SN10 to TNFR1

1 The running buffer flows through the channel set in the CM-5 sensor chip at a flow rate of 10 µl/min until the baseline level is reached.
2 Activated the surface reactive groups of each channel of the chip with the buffer recommended by the instrument.
3 Dissolve TNFR1 and TNFR2 lyophilized powder with EP buffer, inject at a certain concentration, coat it on the surface of the chip, and then block the chip with 1 mol/L ethanolamine. The regeneration conditions are tested prior to the determination of the kinetic curve to select suitable regeneration conditions.
4 When the baseline was stable, a series of peptides were injected and the intermediate concentration of peptides was injected again and the response for each concentration was recorded.

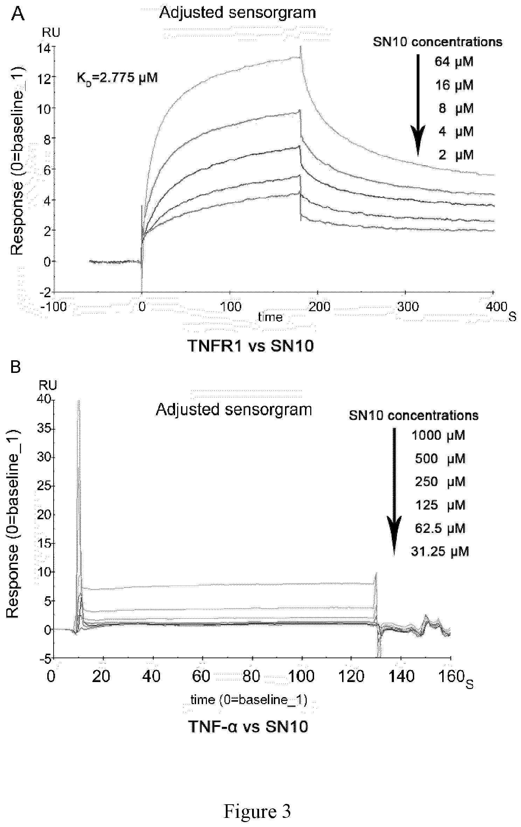

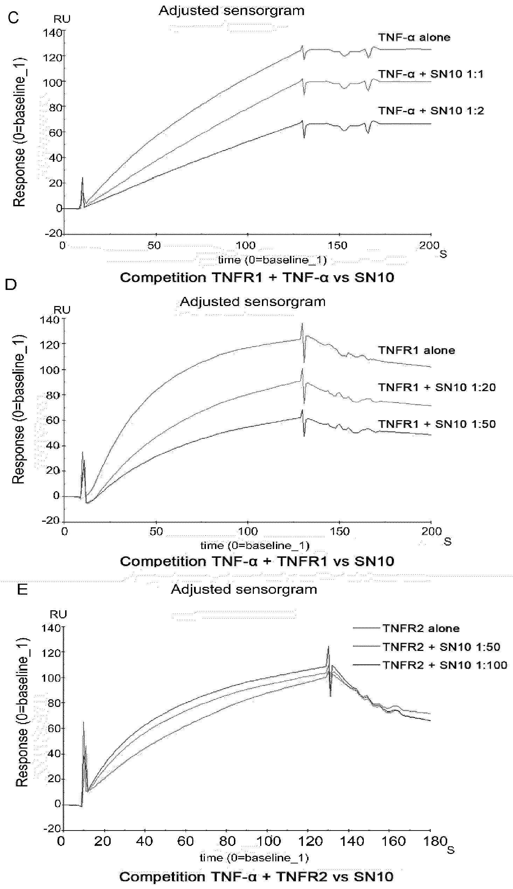

As shown in Figure 3, Hydrostatin-SN10 interacts directly with TNFR1, binding ability with TNFR1 at approximately 2.8 µM; Hydrostatin-SN10 does not bind to TNF-α and competitively inhibits the interaction of TNFR1 with TNF-α.

### Example 3: MST analysis of the binding ability of Hydrostatin-SN10 to TNFR1.

### 1 Interaction of Hydrostatin-SN10 with TNF-α, TNFR1, TNFR2:

Prepare a series of gradient concentrations of Hydrostatin-SN10 in a 1:1 dilution ratio, mix an equal volume of fluorescently labeled TNF-α/TNFR1/TNFR2 at 200nM with Hydrostatin-SN10, incubate in the dark for 30 min, and aspirate the appropriate amount of sample with a capillary pipette to detect. Detected dose-response curve of Thermophoresis and the time trajectory of relative fluorescence values, and calculate the affinity K_{D} value by software NT Affinity Analysis v2.0.2 to determine whether there is a specific binding tendency.

### 2 Competitive inhibition of Hydrostatin-SN10 between TNF-α and TNFR1/TNFR2:

Prepare a series of gradient concentrations of TNF-α in a 1:1 dilution ratio, mix an equal volume of fluorescently labeled TNFR1/TNFR2 at 200nM with TNF-α, incubate in the dark for 30 min, and aspirate the appropriate amount of sample with a capillary pipette to detect. The K_{D} values of the positive control TNFR1/TNFR2 and TNF-α were determined; 400nM TNFR1 and 400µM Hydrostatin-SN10 were mixed in equal volume, and then incubated with a series of concentrations of TNF-α in an equal volume for 30 min, and aspirate the appropriate amount with a capillary pipette to detect. Calculate the K_{D} value by software.The changes of TNF-α saturation concentration, response amplitude and KD value before and after Hydrostatin-SN10 were compared. 3 Competitive inhibition of Hydrostatin-SN10 between TNF-α and TNFR1/TNFR2 (same as method 2).

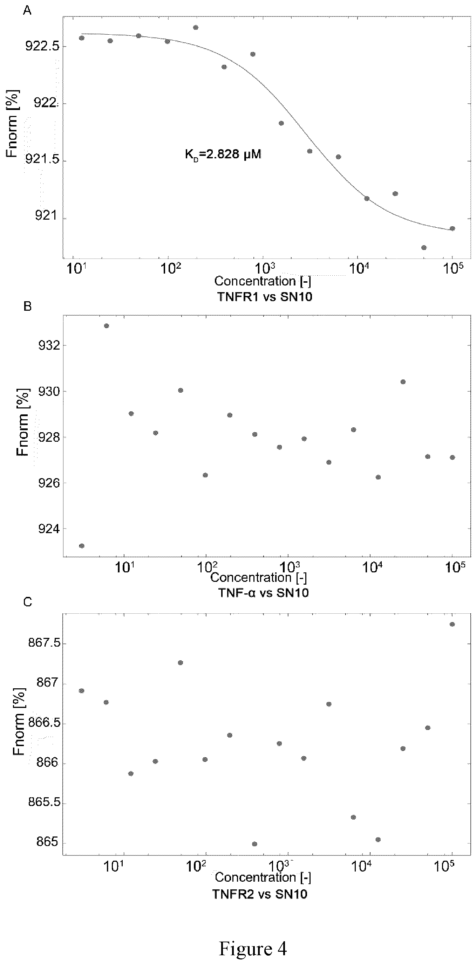

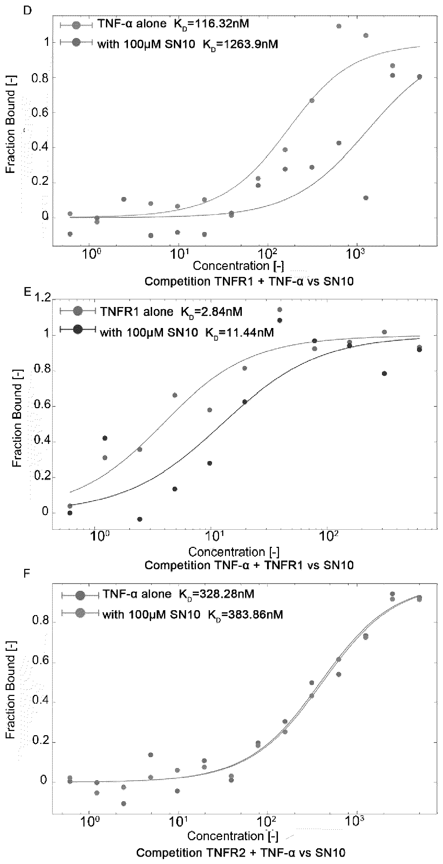

As shown in Figure 4, the results of MST showed that Hydrostatin-SN10 has specific target and directly interact with TNFR1 in a binding capacity of 2.8µM; Hydrostatin-SN10 only binds to TNFR1 and has selectivity, but not binding with TNF-α and TNFR2 ,competitively inhibit the interaction of TNFR1 and TNF-α.

### Example 4: Detection of plasma half-life of Hydrostatin-SN10 and PEG-SN10 in SD rats.

The assay was performed according to the manufacturer's instructions using an ELISA kit purchased from Genzyme. Serum samples were collected from angular vein with heparinized 50µL capillaries, and blood samples were collected at 1 min, 2 min, 3 min, 5 min, 10 min, 15 min, 20 min, 30 min, 45 min, 1 h, 2 h, 4 h, 6 h, 8 h after treatment. After standing for 2 h, the sample was centrifuged, and the obtained supernatant was stored at -20 ° C for testing.

**Table 1**

| Treatment | Plasma half-life(h) |
|---|---|
| Hydrostatin-SN10 | 2.18 |
| PEG-SN10 | 3.79 |

As shown in Table 1, the results show that PEG-SN10 has a longer plasma half-life than Hydrostatin-SN10 after PEG modification.

### Example 5: Treatment effect of Hydrostatin-SN10 and PEG-SN10 in rheumatoid arthritis.

### 1 Experimental animal

DBA/1 mice, male, 8∼10 weeks, weight 18∼20g, 30 mice.

### 2 Main reagents

Type II Bovine Collagen (Chondrex), Complete freund's adjuvant (Chondrex), Incomplete Freund's Adjuvant (Chondrex), Horseradish peroxidase labeled IgG antibody (Ebioscience), Mouse IL-17 ELISA Kit (Ebioscience), LEGENDplex™ multifactor (TNF-α, IL-6, IL-10, IFN-γ) flow detection kit (Biolegend, USA), True-Nuclear™ Mouse Treg Flow™ Kit (FOXP3 Alexa Fluor ® 488 / CD4 APC/CD25 PE) (Biolegend, USA), FITC-Anti-mouse CD3 (Biolegend, USA), PE/Cy7 anti-mouse CD4 (Biolegend, USA), PerCP/Cy5.5 anti-mouse IFN-γ (Biolegend, USA), PE/Dazzle ™ 594 Anti-mouse IL-4 (Biolegend, USA), Alexa Fluor ® 647 anti-mouse IL-17A(Biolegend, USA), FOXP3 Fix/Perm Buffer (4x) (Biolegend, USA).

### 3 The experimental method

After 3 days of adaptation, DBA/1 mice were weighed and began to model. Six were randomly selected as the normal group, and the rest were the experimental group. Day 0: Take bovine type II collagen (4 mg/ml) and vortex and mix with an equal volume of CFA (4 mg/ml). It is better to not disperse on the water, and 100 ul/head is injected intradermally into the tail of the mouse.

Day 21: Take bovine type II collagen (4 mg/ml) and vortex mixed with an equal volume of IFA (4 mg/ml) until the droplets are not dispersed on the water. The mice are injected intradermally with 100 ul/head. On the 30th day, the model was successfully established. The model group (PBS), the administration group-SNIO (1600 ug/kg), the administration group PEG-SN10 (1600 ug/kg), and the infliximab (4 mg/kg) started the abdominal cavity on the same day. The drug was administered by injection once a day for 18 days.

### 4 results

One-Way ANOVA statistical analysis of the data using Graphpad Prism 5 was considered statistically significant at P < 0.05. "*" indicates P < 0.05, "**" indicates P < 0.01, and "***" indicates P < 0.001. 1) The effect of Hydrostatin-SN10 and PEG-SN10 on the score of mouse arthritis index.

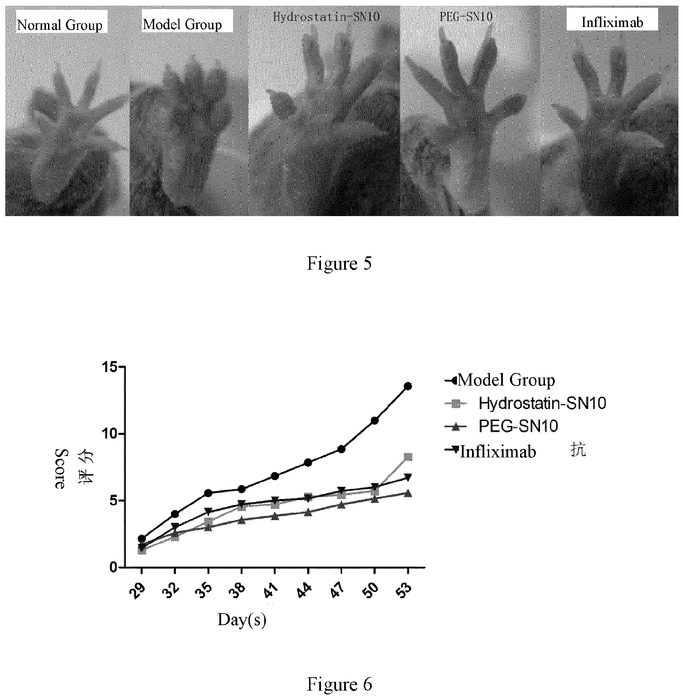

As shown in Figure 5 and Figure 6, after 29 days of initial immunization, the arthritis index of each group gradually increased, that is, the inflammation gradually increased. With the prolongation of time, the swelling of the hind limbs of the mice gradually subsided, but the joints appeared to be stiff or deformed. The highest rating. Compared with the model group, the hydrostatin-SN10 and PEG-SN10 arthritis indexes in the drug-administered group were all reduced, and the disease progression was slowed down.

### 2)Effects of Hydrostatin-SN10 and PEG-SN10 on collagen-specific antibodies and inflammatory factors in serum of CIA mice

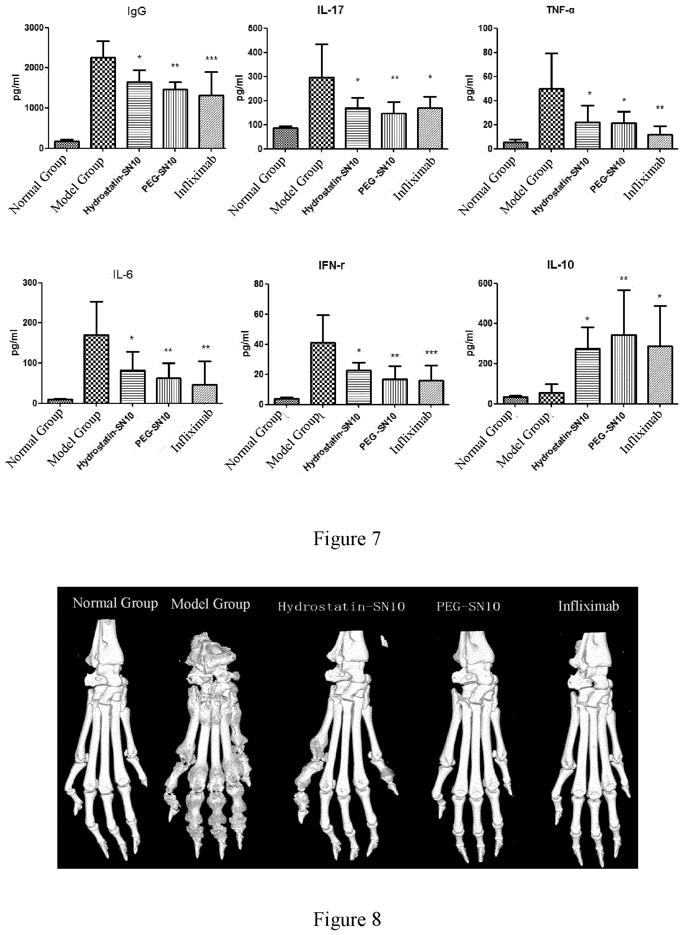

The ELISA kit and multi-factor kit were used to determine the collagen-specific antibody Ig G and pro-inflammatory factors IL-17, TNF-α, IL-6, IFN-γ and anti-inflammatory factor IL-10 in the serum of each group of mice. The content of each group is shown in Figure 7. Compared with the normal control group, the collagen-specific antibody Ig G and the pro-inflammatory factors IL-17, TNF-α, IL-6, IFN-γ in the serum of the model group mice. The content was significantly higher than that of the normal group, and the content of the anti-inflammatory factor IL-10 was not different from that of the normal group. Compared with the model group, the administration groups of Hydrostatin-SN10 and PEG-SN10 significantly reduced the content of collagen-specific antibody Ig G and pro-inflammatory factors IL-17, TNF-α, IL-6 and IFN-γ in serum. Significantly increased the content of the anti-inflammatory factor IL-10.

### 3)Micro-CT detection of the effects of Hydrostatin-SN10 and PEG-SN10 on bone destruction in CIA mice.

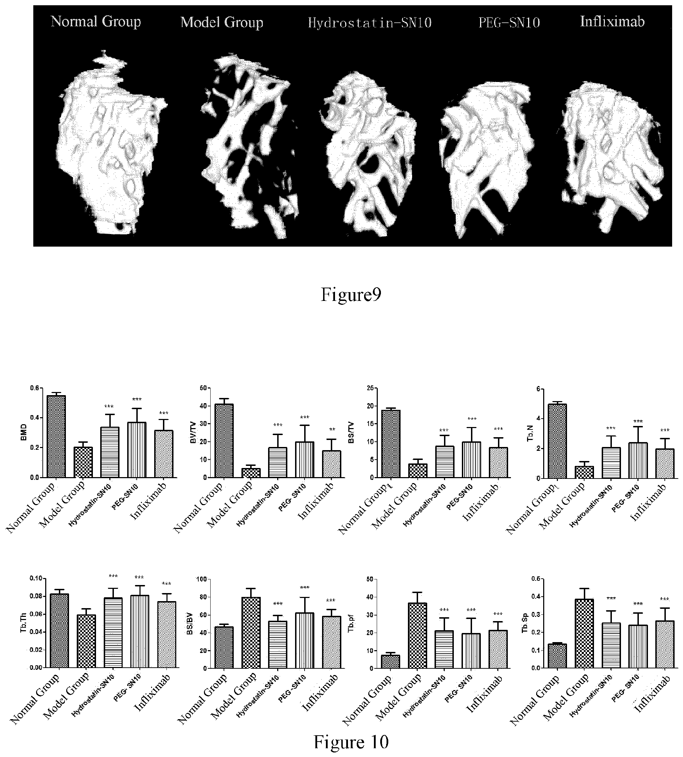

As shown in Figure 8: Micro-CT examination of the hind limbs of the mice showed that the articular surface of the normal group was smooth and the joint structure was clearly visible; the CIA model group had a rough joint surface and severe bone destruction; while the administration group was Hydrostatin-SN10 and PEG-SN10. And the positive drug infliximab significantly reduced the degree of bone destruction. As shown in Figure 9, the mouse trabecular Micro-CT results showed that the normal group of trabecular bone was intact, while the CIA model group had severe bone destruction and significant voids compared with the normal group; compared with the model group, Hydrostatin- SN10, PEG-SN10 and positive drug infliximab have significantly reduced bone damage

As shown in Figure 10, the results of trabecular bone measurements showed: by analysis of bone mineral density (BMD), bone volume/tissue volume (BV/TV), bone area/tissue volume (BS/TV), and number of trabeculae (Th. N), trabecular thickness (Tb.Th), bone surface area/bone volume (BS/BV), trabecular model factor (Tb.pf), trabecular separation (Tb.sp) and other bone parameters, analysis Hydrostatin-SN10, PEG-SN10 relief of bone destruction. The results showed that the model group had bone mineral density (BMD), bone volume/tissue volume (BV/TV), bone area/tissue volume (BS/TV), number of trabeculae (Th.N), trabecular bone compared with the normal group. Parameters such as thickness (Tb.Th) were significantly down-regulated, and parameters such as bone surface area/bone volume (BS/BV), trabecular model factor (Tb.pf), and trabecular separation (Tb.sp) were significantly up-regulated; Bone mineral density (BMD), bone volume/tissue volume (BV/TV), bone area/tissue volume (BS/TV), and number of trabeculae (Th.N) in the Hydrostatin-SN10 and PEG-SN10 groups compared to the model group Parameters such as trabecular thickness (Tb.Th) are significantly up-regulated, parameters such as bone surface area/bone volume (BS/BV), trabecular model factor (Tb.pf), and trabecular separation (Tb.sp) are significant down.

### 4)HE staining observation of the effects of Hydrostatin-SN10 and PEG-SN10 on joint destruction in CIA mice

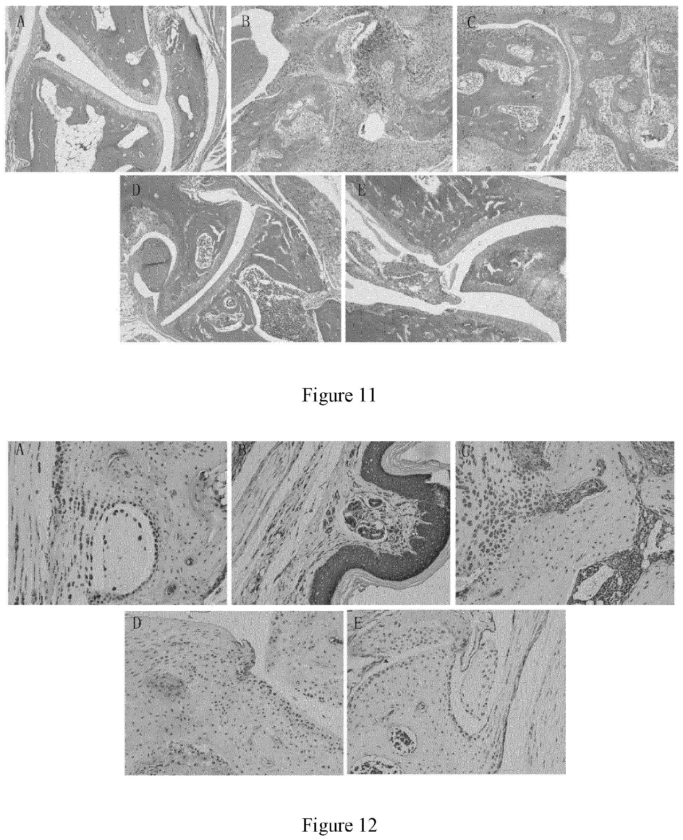

As shown in Figure 11, the HE staining results of the mouse joint showed that the synovial tissue of the joint capsule of the normal group was intact, the fibrous membrane was clearly visible, no exudation was observed in the cystic cavity, and no obvious inflammatory cells were observed in the synovial and synovial tissues. Infiltration; CIA model group showed severe joint structure damage, synovial hyperplasia, severe cartilage damage, severe inflammatory cell infiltration;Hydrostatin-SN10 showed intact joint structure, severe synovial hyperplasia and more severe inflammatory cell infiltration. However, PEG-SN10 and infliximab showed complete joint structure and weak inflammatory cell infiltration, and joint damage was significantly weakened.

### 5) Immunohistochemical observation of the effects of Hydrostatin-SN10 and PEG-SN10 on the expression of TNF-α in CIA mice

Previous results showed that the Hydrostatin-SN10 target was specific and could specifically bind to TNFR1, but not to TNF-α and TNFR2, and Hydrostatin-SN10 competitively inhibited the binding of TNF-α to TNFR1. Thereby reducing the expression of TNF-α, as shown in Figure 12, the expression of TNF-α was increased in the model group compared with the normal group; TNF-α in the joints of Hydrostatin-SN10 and PEG-SN10 compared with the model group. The amount of expression is reduced.

### 6)TRAP staining observation of the effects of Hydrostatin-SN10 and PEG-SN10 on osteoclasts in joints

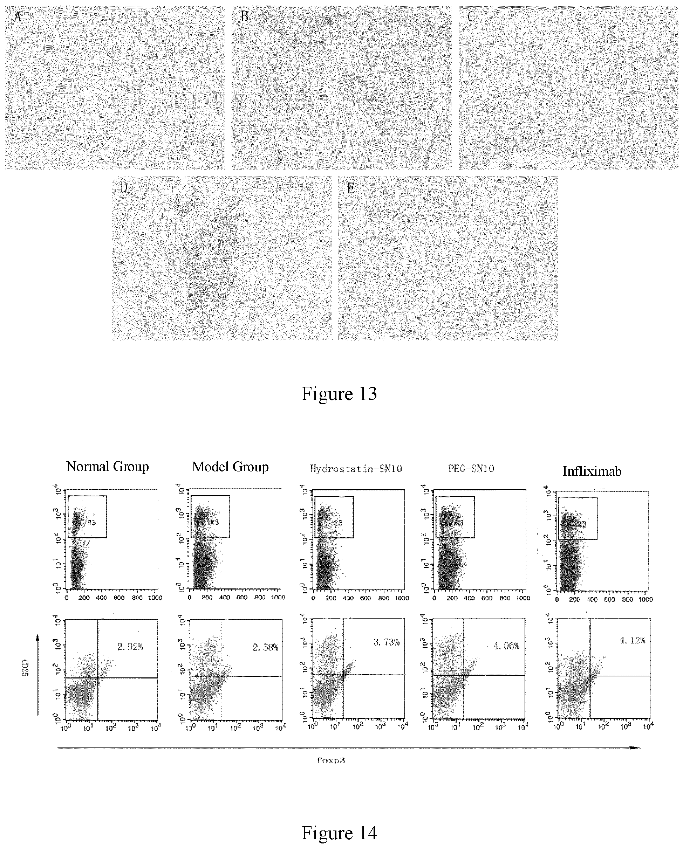

Osteoclasts are a type of bone tissue that performs the function of bone resorption. Osteoclasts correspond to osteoblasts functionally. TRAP, a tartrate-resistant phosphatase, is a specific marker of osteoclasts, and its expression and secretion are closely related to the differentiation and function of osteoclasts. As shown in Figure 13, the anti-tartaric acid phosphatase was significantly increased in the CIA model group compared with the normal group. Compared with the model group, Hydrostatin-SN10 and PEG-SN10 reduced the resistance to tartrate phosphatase and reduced osteoclasts. The amount of this, thereby reducing the erosion of bone tissue.

### 7)Effect of Hydrostatin-SN10 and PEG-SN10 on the expression of Foxp3 in Treg cells of CIA mice

Treg cells are a special type of immunoregulatory cells with two major functional features: immune non-reactivity and immunosuppression. It can inhibit the activity and function of immune cells such as B cells and T cells by direct contact and secretion of inhibitory cytokines between cells, and is extremely important for preventing autoimmune diseases.

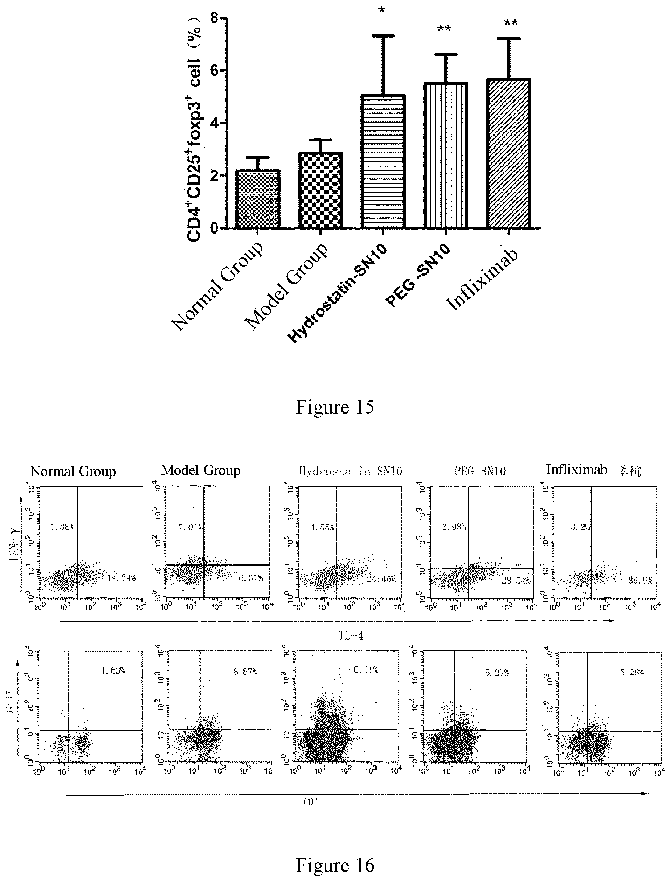

Evidence suggests that there is a change in the number of Treg cells in RA patients, suggesting that Treg cell abnormalities are closely related to the development of RA. Foxp3 is specifically expressed in Treg cells and plays an important role in the proliferation and function of Treg, so Foxp3 can be used as a biomarker of Treg. As shown in Fig. 14 and Fig. 15, compared with the model group, the expression levels of Fxop3 in Hydrostatin-SN10, PEG-SN10 and infliximab were significantly increased, that is, the number of Treg cells was increased, and the immunosuppressive effect was significantly enhanced. Thereby inhibiting the occurrence and development of rheumatoid arthritis.

### 8)Effects of Hydrostatin-SN10 and PEG-SN10 on the expression of Th1, Th2 and Th17 in the spleen of CIA mice

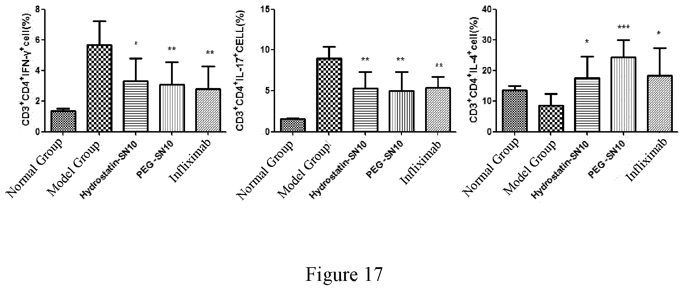

Th1: mainly secretes IFN-γ, mainly mediates cellular immune response, induces the production of inflammatory factors, macrophage activation, etc., mainly induces the production of autoimmune diseases; Th2: mainly secretes IL-4, mainly mediator fluid immunity, Inhibits monocytes and promotes the release of inflammatory cytokines. And the imbalance of Th1/Th2 ratio plays an important role in the pathogenesis of RA. Studies have shown that Th17 cells secrete high levels of IL-17, which stimulates the secretion of matrix metalloproteinase-1, IL-1β by FLS.TNF-a and other co-inducing NF-κB receptor activator ligands are involved in the bone destruction of RA and inflammatory damage in tissues. The effect was observed by measuring the expression of helper T cells in splenocytes. As shown in Figure 16 and Figure 17, compared with the model group, the expression levels of IFN-γ and IL-17 in Hydrostatin-SN10, PEG-SN10 and infliximab were significantly down-regulated, and the expression of IL-4 was up-regulated. That is, Hydrostatin-SN10, PEG-SN10 may be adjusted Changes in Th1, Th2, and Th17 inhibit the expression and release of inflammatory factors and reduce damage to bone.

These results indicate that Hydrostatin-SN10 and PEG-SN10 are effective in the treatment of type II collagen-induced animal models of rheumatoid arthritis in mice.

The embodiments of the present invention have been specifically described above, but the present invention is not limited to the embodiments, and various equivalent modifications can be made by those skilled in the art without departing from the inventive spirit of the present invention. These and other equivalents are intended to be included within the scope of the invention as defined by the appended claims.

## Claims

1. A application of a selective TNFR1 antagonist peptide Hydrostatin-SN10 for the preparation of a medicament for preventing and treating rheumatoid arthritis, wherein the nucleotide sequence of the gene encoding the selective TNFR1 antagonist peptide Hydrostatin-SN10 is SEQ ID NO: 1; the amino acid sequence of the selective TNFR1 antagonist peptide Hydrostatin-SN10 is shown in SEQ ID NO: 2.

2. The application according to claim 1, **characterized in that** the selective TNFR1 antagonist peptide Hydrostatin-SN10 has a molecular weight of 1250.29 Daltons.

3. The application according to claim 1, **characterized in that** the drug for preventing and treating rheumatoid arthritis selectively antagonizes TNFR1

4. The application according to claim 1, **characterized in that** the medicament for preventing or treating rheumatoid arthritis is: the selective active ingredient TNFR1 antagonist peptide Hydrostatin-SN10, or comprises a selective agent Pharmaceutical composition of TNFR1 antagonist peptide Hydrostatin-SN10.

5. The application of a selective TNFR1 antagonist peptide PEG-SN10 based on m PEG2000 modification for the preparation of a medicament for preventing or treating rheumatoid arthritis, **characterized in that** the carboxyl group of m PEG2000 is covalently linked to Hydrostatin- The amino acid sequence of the Hydrostatin-SN10 is shown in SEQ ID NO: 2 on the free amino group of the N-terminal aspartic acid of the SN10 peptide chain.

6. The application according to claim 5, **characterized in that** the mPEG2000 has an average molecular weight of 2000 Daltons.

7. The application according to claim 5, **characterized in that** the medicament for preventing and treating rheumatoid arthritis is: a pharmaceutical composition having PEG-SN10 as the sole active ingredient or a pharmaceutical composition comprising PEG-SN10.

8. The application according to claim 4 or 7, **characterized in that** the pharmaceutical composition and the pharmaceutically acceptable conventional pharmaceutical excipient are formulated into pharmaceutical preparations.

9. The use according to claim 8, wherein the pharmaceutical preparation is tablet, granule, dispersing agent, capsule, soft capsule, dropping pill, injection, powder injection or aerosol.


### SEQUENCE LISTING

<110> Guilin Bajiayi Pharmaceutical Co., Ltd.
<120> A selective TNFR1 antagonist peptide SN10 and its application in rheumatoid arthritis
<130> /
<160> 2
<170> Patent In version 3.3
<210> 1
   <211> 30
   <212> DNA
   <213> Artificial sequence
<400> 1
   gacgaacaac acctagagac cgaactacac 30
<210> 2
   <211> 10
   <212> PRT
   <213> Artificial sequence
<400> 2